# FASCICULE DE BREVET EUROPEEN

(11) **EP 1 310 165 B1**
(45) Date de publication et mention de la délivrance du brevet: **26.10.2005**
(21) Numéro de dépôt: 03075348.7
(22) Date de dépôt: 29.04.1999
(51) Int. Cl.: A01K 61/00, A01K 67/02

(54) **Procédé de reproduction et d'élevage en milieu aquatique notamment de silurus glanis et dispositif permettant de le mettre en oeuvre**
Verfahren zum Züchten von Silurus Glanis in einer wässrigen Umgebung und Vorrichtung zur Durchführung dieses Verfahrens
Method for reproducing and rearing Silurus Glanis in particular in aquatic medium and implementing device

(30) Priorité: 30.04.1998 FR 9805546; 31.12.1998 FR 9816759
(43) Date de publication de la demande: 14.05.2003
(62) Demande divisionnaire de: 99916953.5
(73) Titulaire: Ribes, Gilbert, 34530 Montagnac (FR); Ribes, André, 34530 Montagnac (FR)
(72) Inventeur: Ribes, Gilbert, 34530 Montagnac (FR); Ribes, André, 34530 Montagnac (FR)
(74) Mandataire: Richebourg, Michel François

(56) Documents cités:
- FR-A- 2 187 214
- US-A- 3 024 764
- US-A- 3 765 372
- US-A- 4 182 269
- US-A- 4 214 551
- US-A- 4 489 674
- US-A- 4 697 546
- US-A- 4 742 798

## Description

La présente invention concerne les domaines de l'aquaculture et de la pisciculture et notamment les adaptations et les moyens qui rendent possibles la reproduction et l'élevage, dans des conditions optimales des différentes espèces vivant en milieux aquatiques comme par exemple les poissons, crustacés, mollusques, etc...

Les insuffisances graves des modes de production aquacoles et piscicoles, tels que pratiqués actuellement, sont bien connues. Elles se traduisent par des difficultés aux plans de la qualité et de l'image des productions et ont des conséquences négatives au plan de leur commercialisation. Elles se manifestent principalement au niveau des conduites d'élevage et de la gestion des biotopes correspondants souvent conçues et réalisées, pour la recherche d'une productivité accrue, en contradiction avec les exigences fondamentales de la biologie.

Les contraintes et les dérives résultant de l'intensification en matière de productions aquacoles et piscicoles conduisent couramment à confiner - avec des densités importantes - les sujets élevés dans des bassins de type artificiel- en béton ou en matières "plastiques", etc...- ou bien dans des cages immergées en étangs ou en mer, les privant ainsi du contact normal avec l'adversité naturelle que doit affronter l'espèce concernée, notamment aux niveaux bactériens, parasitaires, etc..

La mise en place et le développement des défenses immunitaires s'en trouvent fortement perturbés ou inhibés chez les sujets concernés, créant ainsi une situation de dépendance par rapport aux moyens de la pharmacie vétérinaire et plus particulièrement, aux antibiotiques souvent incorporés directement lors de leur fabrication dans la composition des aliments habituellement distribués.

Cette situation de dépendance est, de surcroît, aggravée par le recours systématique à une alimentation entièrement artificielle dont la production industrielle ne peut assurer une parfaite continuité des données qualitatives, ni éviter, de ce fait, l'apparition de carences souvent à l'origine des défaillances du système immunitaire ou de leur aggravation.

Ces aspects négatifs des formes conventionnelles, et notamment intensives, d'élevage aquacole et piscicole régulièrement constatés dans les phases de grossissement des alevins et des juvéniles vers leurs tailles commerciales prennent une signification beaucoup plus importante encore, par leurs conséquences, dans les phases de reproduction et d'élevage larvaire.

En effet, les gamètes de géniteurs retenues parmi les sujets élevés suivant les modes conventionnels décrits antérieurement n'ont pas le niveau élevé de qualité biologique des gamètes de géniteurs vivant dans les conditions naturelles ou dans des conditions de type naturel, notamment au niveau du vitellus des ovocytes.

L'embryogénèse et la vie larvaire en sont fortement affectées conduisant, dans cette situation également, à un recours très précoce, c'est à dire dès la phase d'incubation, aux moyens de la pharmacie vétérinaire. Cette situation entraîne une fragilisation des nouveaux sujets, initiée dès l'oeuf et l'embryon, créant ainsi les conditions d'une déficience définitive.

En outre, certains aspects des protocoles de reproduction couramment employés notamment pour les poissons d'eau douce accentuent encore l'état initial biologiquement déficient de l'embryon et de la larve.

En effet, à l'occasion des manipulations de fécondation et d'incubation, pour éviter que les oeufs ne se collent entre eux et ne s'agglutinent -ce qu'ils feraient naturellement mais les conduirait pour la plupart à périr par défaut d'oxygénation- il est pratiqué leur "décollage" au moyen, notamment, d'enzymes protéolytiques additionnés à l'eau de l'incubateur.

Ces produits cumulent leurs effets avec ceux des fongicides, anti-parasitaires, bactéricides, utilisés couramment d'une manière conventionnelle en cours d'incubation. Il en résulte une détérioration plus ou moins importante suivant les doses employées de la membrane des oeufs, décollés et en mouvement dans l'incubateur, au moment où son rôle notamment dans l'oxygénation de l'embryon doit être, au contraire, favorisé.

En outre, les oeufs décollés flottent dans l'incubateur où ils sont inutilement en continuelle agitation; leur contrôle, comme par exemple l'observation de l'embryogénèse en cours, est alors très difficile sinon impossible.

Par contre en aquaculture semi-artificielle, il est parfois prévu de placer dans les zones de frai, des nids confectionnées en branchages afin de récupérer les oeufs; nids pouvant être ensuite placés dans les incubateurs.

Une des méthodes ayant pour but de potentialiser les résultats de l'aquaculture, notamment en matière de croissance des sujets élevés pour leur commercialisation, consiste précisément à rendre impossible les activités de reproduction et donc d'empêcher les maturations successives après la puberté.

Un des moyens permettant de rendre impossibles les activités de reproduction des sujets élevés, obtenu pour les salmonidés, est de les rendre triploïdes.

La triploïdie qui existe naturellement de manière exceptionnelle dans les milieux aquatiques correspond à la présence dans le noyau de la cellule de trois lots chromosomiques identiques -chacun représentant le patrimoine génétique du sujet- au lieu des deux lots chromosomiques identiques qui définissent la diploïdie, situation normale de la cellule.

La triploïdie n'est pas une manipulation génétique; le lot chromosomique représentant le patrimoine génétique n'est pas affecté et reste identique qu'un même sujet soit triploïde ou diploïde. Le sujet triploïde est donc normalement sexué mais il est stérile.

En l'absence d'activité de reproduction, la totalité de l'énergie consommée par le sujet élevé sera consacrée à sa croissance qui sera donc beaucoup plus rapide que celle du même sujet diploïde : la productivité de l'aquaculture s'en trouve fortement accrue.

La triploïdie permet également, par la stérilité qui en résulte, de maîtriser totalement la démographie de l'espèce concernée; elle rend ainsi possible son introduction dans le milieu naturel notamment à des fins écologiques, sans courir le risque de perdre le contrôle de sa population et d'une prolifération dangereuse; cela prend une signification particulière lorsqu'il s'agit, par exemple, d'un carnassier de très grande taille, comme Silurus Glanis.

En outre, la triploïdie supprime les risques de pollution génétique et autorise l'utilisation de l'espèce concernée à des fins écologiques ou d'élevage aquacole dans des zones où elle n'est pas naturellement présente et où elle ne fait donc pas partie des écosystèmes existants. Dans toutes ces situations, les nécessités de la lutte pour la préservation des espèces naturelles et pour la sauvegarde des écosystèmes conduisent à des attitudes administratives et à des réglementations interdisant l'introduction et l'utilisation des sujets diploïdes de l'espèce concernée étrangère. Dans le cas de Silurus Glanis par exemple, naturellement présent dans les parties continentales de la zone tempérée en Europe et en Asie occidentale, la triploïdie de l'espèce en permet l'introduction et l'utilisation, techniquement et administrativement, dans d'autres zones climatiques, par exemple tempérées chaudes, subtropicales, tropicales, équatoriales, etc...

Une des méthodes connues pour obtenir la triploïdie consiste à appliquer un choc thermique à l'oeuf; ce choc thermique doit être réalisé dans des conditions particulières, à un moment précis, à une température donnée et durant un temps déterminé.

Une des conséquences du choc thermique est de détériorer relativement l'oeuf traité notamment au niveau du vitellus et d'avoir, de ce fait, un effet négatif sur l'embryogénèse.

Les conditions de maturation, de reproduction, d'incubation en aquaculture conventionnelle amplifient considérablement ces difficultés, jusqu'à rendre la triploïdie inaccessible pour la plupart des espèces commerciales - si l'on excepte les salmonidés - et à ne l'obtenir qu'avec des taux de triploïdie aléatoires par rapport aux sujets traités.

C'est donc dans une sorte de spirale négative que les insuffisances de l'activité aquacole et piscicole conventionnelle se constatent en se renforçant mutuellement tant au niveau des phases de grossissement des alevins et des juvéniles vers leurs tailles commerciales qu'au niveau de la génération -maturations des géniteurs, reproduction, élevage larvaire- des futurs sujets destinés au grossissement et à la poursuite de la génération elle-même.

Un exemple de procédé d'élevage et de reproduction en milieu aquatique est décrit dans le brevet américain n° 3,765,372 qui ne fait pas mention d'un support d'oeufs susceptible de réaliser le déplacement de ces derniers. Il décrit toutefois, la possibilité de récupérer à la surface de l'incubateur les oeufs flottants et d'évacuer du fond de l'incubateur les oeufs morts. Il n'est pas non plus décrit que les oeufs sont déplacés à partir de l'incubateur.

Le document américain n° 4,742,798 au nom de Blackett décrit un incubateur adoptant une plaque percée statique, disposée à mi-hauteur sur laquelle peuvent être déposés des substrats support d'oeufs également ajourés de façon à non seulement servir de supports à des oeufs non collants déposés à leur surface mais également favoriser leur oxygénation facilitant par ce fait, leur incubation. Néanmoins, les substrats sont constitués par de nombreuses formes alvéolées indépendantes les unes des autres susceptibles de servir de supports à un ou plusieurs oeufs mais ne pouvant pas faire l'objet d'une amovibilité globale, c'est à dire synchronisée pour l'ensemble des substrats. Ces substrats qui sont destinés à plus ou moins reproduire le support sur lequel est susceptible de pondre certaines espèces de poissons en milieu naturel tels les galets, reposent de par leur poids sur la plaque percée. De plus, la plaque percée permettant de réguler le flux de liquide à l'intérieur de l'incubateur n'est pas conçue pour être retirée et replacée en position. Il est même prévu que cette plaque soit soudée aux bords de l'incubateur.

La présente invention apporte une solution aux insuffisances de l'art ancien (ou à la pratique conventionnelle) principalement sur les points décrits. Elle y parvient en évitant ou en supprimant les contradictions entre les procédés d'élevage et les contraintes naturelles ordinaires de la biologie, et au contraire en développant, en normalisant et en potentialisant les moyens naturels dont la nature ne dispose souvent que d'une manière discontinue et/ou aléatoire.

Le procédé de reproduction et d'élevage de l'invention sur la base d'oeufs susceptibles d'être collés et fixés est du type de celui comprenant les phases classiques suivantes :
- éclosion, résorption vésiculaire, différents stades de l'élevage larvaire, élevage jusqu'au stade d'alevin ou de juvénile en écloserie,
- croissance et grossissement en bassins,
- passage de la phase alevin ou de la phase juvénile à la phase adulte,
- sélection des géniteurs, maturation,
- prélèvement des ovocytes et des spermatozoïdes,
- fécondation,
- embryogénèse en incubateur.

Selon la caractéristique principale de l'invention, le procédé d'aquaculture est remarquable en ce qu'il consiste à placer dans l'incubateur, avant l'introduction des oeufs, un support amovible les accueillant sur des zones de réception permettant aux oeufs de s'y fixer au moment de leur introduction dans l'incubateur, lequel support permet, à un même moment précis, le déplacement de la totalité des oeufs.

Ce support a pour avantage de permettre la fixation individuelle des oeufs qui sont ainsi immobilisés pendant toute la durée de l'embryogénèse chacun étant parfaitement exposé à la circulation de l'eau dans l'incubateur et de ce fait bénéficiant notamment d'un oxygénation optimale.

La fixation individuelle des oeufs sur le support évite qu'ils ne s'agglutinent entre eux et de ce fait périssent pour la plupart par insuffisance d'oxygénation et rend inutile l'addition à l'eau de l'incubateur de produits, tels les enzymes protéolytiques, destinés précisément en aquaculture conventionnelle à les séparer après qu'ils se soient agglutinés.

En outre, les oeufs étant fixés et immobilisés et non plus inutilement en mouvement pendant toute la durée de l'incubation, l'embryogénèse pourra être observée et contrôlée en continu, notamment dans sa phase finale.

L'association entre les brevets américains n°3,765,372 et n°4,742,798 ne rend pas intellectuellement évidente cette caractéristique en ce que la matière d'oeuvre traitée à savoir les oeufs des espèces élevés diffèrent grandement. En effet, le procédé proposé dans le document n°3,765,372 est un procédé d'élevage concernant des espèces marines dont les oeufs flottent et ne se déposent pas. Or, le dispositif décrit dans le document n°4,742,798 est spécifiquement conçu pour des oeufs de salmonidés qui ne flottent pas mais se déposent gravitairement sur les substrats prévus à cet effet et objet de l'invention décrite dans ce document. En conséquence, l'association de ces deux documents est non seulement non évidente mais impossible pour l'homme de l'art qui connaît les caractéristiques des oeufs.

Contrairement à l'art antérieur représenté par le document américain n°US-A-4,742,798 dans lequel le dispositif est formé de substrats indépendants les uns des autres, incapables de ce fait, de réaliser un déplacement global sans dommage à un moment précis des oeufs, le support amovible de l'invention assure ce déplacement qui peut avoir plusieurs fonctions.

Une autre caractéristique particulièrement intéressante du procédé de l'invention, consiste à retirer de l'incubateur le support amovible sur lequel sont fixés individuellement les oeufs de manière à interrompre le processus d'incubation à un moment déterminé et éviter de ce fait l'éclosion de larves dont l'embryogénèse aurait une durée trop longue.

En effet, la durée de l'embryogénèse à l'intérieur d'une opération d'incubation est plus ou moins variable selon les oeufs et les larves dont l'embryogénèse aurait une durée trop longue sont moins performantes. Leur élimination, dès avant l'éclosion, permet la sélection des sujets les plus performants et limite fortement la dispersion des tailles et conduit à des lots de production beaucoup plus homogènes, améliorant ainsi significativement la productivité aux plans quantitatifs et qualitatifs.

Une autre caractéristique particulièrement intéressante du procédé de l'invention consiste à placer ledit support amovible, après son retrait de l'incubateur, dans une zone de température différente de manière à provoquer un choc thermique sur les oeufs avant la première division cellulaire; l'application d'un choc thermique, différent suivant l'espèce concernée, permet d'obtenir des sujets triploïdes. Selon un exemple non limitatif de réalisation, pour Silurus Glanis, il s'agit d'un choc froid.

Le support amovible a pour avantage de permettre, à un même moment précis, le déplacement de la totalité des oeufs vers la zone de température différente et d'assurer l'exposition au choc thermique d'une durée identique pour l'ensemble des oeufs et chacun d'entre eux.

Ainsi, une fois définis, les trois paramètres variant en fonction de l'espèce élevée et traitée que sont le temps (ou moment) d'intervention, la température du choc thermique et la durée d'application dudit choc seront identiques pour tous les oeufs et chacun d'entre eux. Bien entendu, l'objectif de l'homogénéité du traitement en amont et durant la triploïdie est de produire cent pour cent de sujets triploïdes, sans risques de diploïdie accidentelle qui reposerait le problème de pollution génétique et de gestion de l'élevage.

Ainsi, le support amovible présente plusieurs avantages selon l'embryogénèse désirée.

Dans le cadre d'une embryogénèse "normale", le support amovible permet :
- une bonne oxygénation,
- un contrôle visuel en continu en l'embryogénèse,
- une maîtrise de la durée de l'embryogénèse permettant une sélection efficace notamment de géniteurs.

Dans le cadre d'une embryogénèse soumise à choc thermique, ledit support amovible permet, en outre :
- l'homogénéisation du traitement thermique, et
- la maîtrise de l'ensemble des étapes de cette opération permettant là aussi, une sélection efficace des sujets les plus performants pour leur élevage.

L'invention concerne également le dispositif permettant de mettre en oeuvre ce procédé et remarquable en ce qu'il comporte un support amovible constitué par un treillis rigide formé d'une armature dont la forme générale s'adapte à la forme intérieure d'un incubateur de façon à pouvoir y être introduit, ledit treillis rigide permettant aux oeufs de s'y fixer individuellement au moment de leur introduction dans l'incubateur et pouvant être retiré de l'incubateur et remis.

Selon une caractéristique particulièrement avantageuse de l'invention, le treillis est formé par deux armatures circulaires liées entre elles par des montants verticaux de façon à ce que les centres des deux cercles soient sur le même axe formant un châssis rigide les montants verticaux et les cercles servant de support et de point d'attache à des fils s'entrecroisant et passant par chaque montant de façon à réaliser un treillage condensé à l'intérieur du châssis permettant aux oeufs de s'y fixer.

Le choix d'un fil comme support à la fixation des oeufs est particulièrement avantageux en ce qu'il permet une fixation sur une petite partie de la membrane, le reste de la surface sphérique de l'oeuf en assurant l'oxygénation.

Selon une caractéristique non limitative de l'invention, les fils constituant le treillage sont de diamètre inférieur au diamètre des oeufs auxquels ils servent de support.

Selon une autre caractéristique particulièrement avantageuse de l'invention, le châssis rigide ainsi que les fils sont en matériau imputrescible conduisant de façon optimale la température à laquelle ils ont été soumis afin de garantir une très grande homogénéité dans le traitement thermique que l'on fait subir aux oeufs fixés au support.

Bien entendu, ledit treillis rigide est muni d'un organe de préhension lui permettant d'être retiré de l'incubateur, placé dans la zone de température différente et remis dans l'incubateur manuellement ou par un quelconque moyen de levage.

On comprend que le procédé et le dispositif qui viennent d'être ci-dessus décrits, l'ont été en vue d'une divulgation plutôt que d'une limitation. Bien entendu, divers aménagements, modifications et améliorations pourront être apportés, sans pour autant sortir du cadre de l'invention définie par les revendications. Ainsi, le biotope auquel s'applique le procédé ne se limite pas à l'eau douce mais à toutes les situations où la vie aquatique est possible.

## Revendications

1. Procédé de reproduction et d'élevage en milieu aquatique sur la base d'oeufs susceptibles d'être fixés du type de celui comprenant les phases classiques suivantes :
- éclosion, résorption vésiculaire, différents stades d'élevage larvaire, élevage jusqu'au stade d'alevin ou de juvénile en écloserie,
- croissance et grossissement en bassins,
- passage de la phase alevin ou de la phase juvénile à la phase adulte,
- sélection des géniteurs, maturation,
- prélèvement des ovocytes et des spermatozoïdes,
- fécondation,
- embryogénèse en incubateur, **CARACTÉRISÉ EN CE QU'**il consiste à placer dans l'incubateur, avant l'introduction des oeufs, un support amovible les accueillant sur des zones de réception permettant aux oeufs de s'y fixer individuellement au moment de leur introduction dans l'incubateur, lequel support permet, à un même moment précis, le déplacement de la totalité des oeufs.

2. Procédé selon la revendication 1, **CARACTÉRISÉ EN CE QU'**il consiste à retirer de l'incubateur le support amovible sur lequel sont fixés individuellement les oeufs de manière à interrompre le processus d'incubation à un moment déterminé et éviter de ce fait l'éclosion de larves dont l'embryogénèse aurait une durée trop longue.

3. Procédé selon la revendication 1, **CARACTÉRISÉ EN CE QU'**il consiste à placer ledit support amovible, après son retrait de l'incubateur, dans une zone de température différente de manière à provoquer un choc thermique sur les oeufs avant la première division cellulaire.

4. Dispositif permettant de mettre en oeuvre le procédé selon l'une quelconque des revendications 1 à 3, comportant un incubateur et un support amovible constitué par un treillis rigide formé d'une armature servant de support et de point d'attache à des fils s'entrecroisant de façon à réaliser un treillage condensé et dont la forme générale s'adapte à la forme intérieure de l'incubateur de façon à pouvoir y être introduit, ledit treillis rigide permettant aux oeufs de s'y fixer individuellement au moment de leur introduction dans l'incubateur et pouvant être retiré de l'incubateur et remis.

5. Dispositif selon la revendication 4, **CARACTÉRISÉ PAR LE FAIT QUE** le treillis est formé par deux armatures circulaires liées entre elles par des montants verticaux de façon à ce que les centres des deux cercles soient sur le même axe formant un châssis rigide les montants verticaux et les cercles servant de support et de point d'attache à des fils s'entrecroisant et passant par chaque montant de façon à réaliser un treillage condensé à l'intérieur du châssis permettant aux oeufs de s'y fixer.

6. Dispositif selon la revendication 4, **CARACTÉRISÉ PAR LE FAIT QUE** le châssis rigide ainsi que les fils sont en matériau imputrescible conduisant de façon optimale la température à laquelle ils ont été soumis afin de garantir une très grande homogénéité dans le traitement thermique que l'on fait subir aux oeufs fixés au support.

7. Dispositif selon la revendication 4, **CARACTÉRISÉ PAR LE FAIT QUE** les fils constituant le treillage sont de diamètre inférieur au diamètre des oeufs auxquels ils servent de support.

## Patentansprüche

1. Verfahren zur Reproduktion und Aufzucht im Gewässer auf der Grundlage von anhaftenden Eiern, in der Art eines Verfahrens, das die folgenden klassischen Phasen aufweist:
- Ausschlüpfen, vesikuläre Resorption, verschiedene Aufzuchtstadien der Larven, Aufzucht bis zum Stadium des Setzlings oder Jungtiers im Zuchtbetrieb,
- Wachstum und Gewichtszunahme in Becken,
- Übergang von der Setzling- oder Jungtierphase in die Erwachsenenphase,
- Auswahl von Zuchttieren, Reife,
- Entnahme von Ei- und Samenzellen,
- Befruchtung,
- Embryogenese im Inkubator, **dadurch gekennzeichnet, dass** vor Einlage der Eier eine bewegliche Auflage in den Inkubator eingesetzt wird, die diese auf Aufnahmebereichen aufnimmt, die es den Eiern gestattet, dort einzeln in dem Augenblick anzuhaften, in dem sie in den Inkubator eingeführt werden, wobei mit dieser Auflage zu einem bestimmten Augenblick alle Eier gemeinsam bewegt werden können.

2. Verfahren nach Anspruch 1, **dadurch gekennzeichnet, dass** die bewegliche Auflage, auf der die Eier einzeln anhaften, aus dem Inkubator genommen werden kann, so dass der Inkubationsprozess in einem bestimmten Augenblick unterbrochen werden kann und deswegen das Schlüpfen der Larven vermieden wird, deren Embryogenese zu lange dauern würde.

3. Verfahren nach Anspruch 1, **dadurch gekennzeichnet, dass** die bewegliche Auflage nach ihrer Entnahme aus dem Inkubator in einen anderen Temperaturbereich verbracht wird, so dass die Eier vor der ersten Zellteilung einem thermischen Schock ausgesetzt werden.

4. Vorrichtung zur Durchführung des Verfahrens nach einem der Ansprüche 1 bis 3, die einen Inkubator und eine bewegliche Auflage umfasst, die aus einem starren Gitter besteht, das von einem Gerüst ausgebildet wird, welches für sich kreuzende Drähte als Auflage und Befestigungspunkt dient, so dass ein dichtes Gitter entsteht, dessen allgemeine Form der Innenform des Inkubators angepasst ist, so dass es dort eingeführt werden kann, wobei die Eier im Augenblick ihrer Einführung in den Inkubator auf dem starren Gitter einzeln anhaften und aus dem Inkubator herausgenommen und wieder zurückgestellt werden können.

5. Vorrichtung nach Anspruch 4, **dadurch gekennzeichnet, dass** das Gitter von zwei kreisförmigen Gerüsten gebildet wird, die miteinander durch senkrechte Holme in einer Art und Weise verbunden werden, dass die Mittelpunkte der beiden Kreise auf derselben Achse liegen und einen starren Rahmen bilden, wobei die vertikalen Holme und die Kreise als Auflage und Befestigungspunkt für die sich kreuzenden und durch jeden Holm verlaufenden Drähte dienen, so dass innerhalb des Rahmens ein dichtes Gitter entsteht, auf dem die Eier anhaften können.

6. Vorrichtung nach Anspruch 4, **dadurch gekennzeichnet, dass** der starre Rahmen sowie die Drähte aus einem unverweslichen Werkstoff bestehen, der die Temperatur, der sie ausgesetzt sind, optimal leitet, um den an der Auflage anhaftenden Eiern eine sehr hohe Homogenität bei ihrer thermischen Behandlung zu gewährleisten.

7. Vorrichtung nach Anspruch 4, **dadurch gekennzeichnet, dass** die das Gitter bildenden Drähte einen kleineren Durchmesser haben als die Eier, denen sie als Auflage dienen.

## Claims

1. A method for reproduction and breeding in an aquatic medium, based on eggs capable of being fixed, of the type of the one comprising the following conventional phases:
- hatching, yolk resorption, different stages of larval breeding, breeding up to the fry or juvenile stage in a hatchery,
- transition from the fry or juvenile stage to the adult stage,
- selection of the spawners, maturation,
- collection of ovoaytes and spermatozoa,
- fertilization,
- embryogenesis in an incubator, **characterized in that** it consists of placing in the incubator, before introducing the eggs, a removable support accommodating them on receiving areas allowing the eggs to be individually fixed thereon when introducing them into the incubator, which support allows the totality of the eggs to be moved at a same precise moment.

2. The method according to claim 1, **characterized in that** it consists of withdrawing from the incubator the removable support on which the eggs are individually fixed, so as to interrupt the incubation process at a determined moment and consequently avoid hatching of larvae for which embryogenesis would have a too long duration.

3. The method according to claim 1, **characterized in that** it consists of placing said removable support, after its withdrawal from the incubator, in an area of different temperature so as to cause a thermal shock on the eggs before the first cell division.

4. A device with which the method according to any of the claims 1 to 3 may be applied, including an incubator and a removable support consisting of a rigid wire netting formed with a frame used as a support and attachment point for intertwining wires so as to achieve a condensed trellis and the general shape of which is adapted to the inner shape of the incubator so that it may be introduced therein, said stiff wire netting allowing the eggs to be fixed thereon when they are introduced into the incubator, and which may be withdrawn from the incubator and put back therein.

5. The device according to claim 4, **characterized by** the fact that the wire netting is formed by two circular frames connected to each other by vertical uprights so that the centers of both circles are on the same axis forming a rigid chassis, the vertical uprights and the circles being used as a support and attachment point for intertwining wires and passing through each upright so as to achieve a condensed trellis inside the chassis allowing the eggs to be fixed thereon.

6. The device according to claim 4, **characterized by** the fact that the rigid chassis as well as the wires are in an imputrescible material optimally conducting the temperature to which they were submitted, in order to guarantee very large homogeneity in the heat treatment to which the eggs fixed to the support are submitted.

7. The device according to claim 4, **characterized by** the fact that the wires forming the trellis are of a smaller diameter than the diameter of the eggs for which they are used as a support.
